# EUROPEAN PATENT SPECIFICATION

(11) **EP 0 358 717 B1**
(45) Date of publication and mention of the grant of the patent: **16.04.1997**
(21) Application number: 88905089.4
(22) Date of filing: 12.05.1988
(51) Int. Cl.: A61B 6/00

(54) **RADIOLOGICAL APPARATUS FOR MEASURING BONE DENSITY**
RÖNTGENGERÄT ZUR MESSUNG DER KNOCHENDICHTE
APPAREIL RADIOLOGIQUE PERMETTANT DE MESURER LA DENSITE OSSEUSE

(30) Priority: 15.05.1987 US 50726
(43) Date of publication of application: 21.03.1990
(73) Proprietor: MEDICAL & SCIENTIFIC ENTERPRISES, INC., Sudbury, MA 01776 (US)
(72) Inventor: O'NEIL, William, Ann Arbor, MI 48104 (US); WARNE, James, R., Washington, PA 15301 (US)
(74) Representative: Greenwood, John David
(86) International application number: US8801576
(87) International publication number: WO8808688

(56) References cited:
- WO-A-86/07531
- DE-A- 2 412 161
- US-A- 3 803 417
- US-A- 3 944 830
- US-A- 4 365 343
- publication LUNAR DP3, Technical Manual Dual-Photon Scanner

## Description

### Background of the Invention

This invention relates to radiologic measuring devices, and more particularly, to the use of radiation in measuring bone structure.

The diagnostic use of radiation in evaluating bone structure has recently been applied in assessing bone demineralization that occurs with advancing age. Bone mineral is lost from all parts of the skeleton, and at a linear rate from the lumbar spine, starting at about 35 years of age. The resultant demineralization results in a high risk of fractures with an increased associated mortality and morbidity. In evaluation of the spine, there is a very good correlation between dual photon densitometry measurements of bone density and fracture resistance in excised vertabrae subjected to compression testing. It is also important to evaluate mineral loss in the hip, as appendicular losses often match or exceed spine loss in patients over 70.

Dual photon absorptiometry enables non-invasive quantitative analysis of bone mineral in regions of the body that were previously inaccessable using single photon absorptiometry. The use of two photon energies minimizes errors that result from irregular body contour and soft tissue inhomogeneities. Essentially, two photon energies are necessary to allow discrimination of two substances of a given system. In this case between bone mineral and soft tissue. The most commonly used photon energies in dual photon scanning are 44 and 100 KeV. The measurements of the attenuation of this radiation as it passes through the body yields the bone mineral density.

An apparatus and method for measuring bone density according to the preamble of claims 1 and 18 described in a publication titled "Lunar DP3 Technical Manual Dual-Photon Scanner" by the firm Lunar. The present invention is characterised by the features of the characterising portions of claim 1 and 18.

### Summary of the Invention

The present invention involves the multidirectional measurement of human bone densities for diagnostic purposes. A radiation source, and a detector used for measuring the radiation transmitted through the object being measured, are rigidly aligned by a bracket or arm. This detector is mounted in a telescoping mechanism to permit control over the source/detector distance. The arm and the attached source and detector, are mounted on an "x-y" table that permits scanning of objects over a predetermined planar area. This apparatus is mounted so that the source, detector, and scanning mechanism can be rotated to view a stationary object from different angles.

In a preferred embodiment of the invention, the pivot axis about which the arm rotates is displaceable so that the source will clear the table upon rotation. The rotating apparatus may be mounted in a drawer with guides or rails that telescope out to support the system during rotation. The rotating elements are weighted so that very little pressure is necessary to rotate the system. The weight is distributed so that if the mechanism is stopped at any point during rotation, it will at most slowly accelerate under its own weight. If the center of gravity of the rotating mechanism is approximately along the pivot axis, this condition will be met. One weight is placed in the detector to vertically adjust the center of gravity. A second weight is placed adjacent the scanning assembly to horizontally adjust the center of gravity.

### Brief Description of the Drawings

Figure 1 is a perspective view of the bone densitometer.

Figure 2 is a side view of the radiation source and detector in the interior position.

Figure 3 is a side view of the apparatus rotated to the lateral position.

Figure 4 is a top view of the saddle and drawer assembly.

### Detailed Description of the Invention

Existing scanner assemblies used in bone densitometry generally permit unidirectional scanning of patients only. To obtain lateral or side views, for example, the patient must be turned. This movement of the patient is often difficult or impossible depending upon their physical condition.

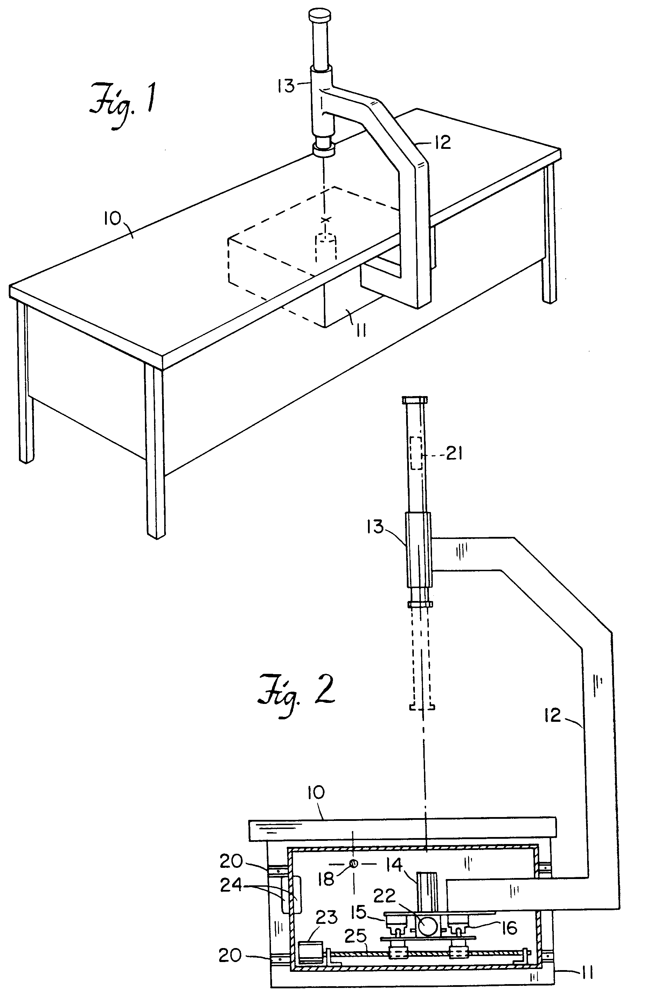

A dual photon bone densitometer used in diagnosing osteoporosis is illustrated generally in Figure 1. A table 10 on which the patient lies has a drawer assembly 11 which is pulled out from under the table on the side from which a bracket 12 protrudes. The bracket 12 extends in a "C" shape from the drawer assembly 11 to a detecting apparatus 13. Figure 2 shows, in a cross-sectional view, the relationship between the detector 13 and the contents of the drawer assembly 11.

A radiation source 14 is mounted on a moveable platform 15. The source 14 is rigidly aligned with the detector 13 by bracket 12 to insure that radiation emitted from the source is received by the detector regardless of the angle to which the source-detector axis is rotated. The entire rotatable apparatus is mounted on a tray or "saddle" 17. The saddle 17 is rotatably mounted onto the assembly plates 19. The plates 19 in one embodiment constitute the side walls of a drawer which compactly houses the source and scanning apparatus.

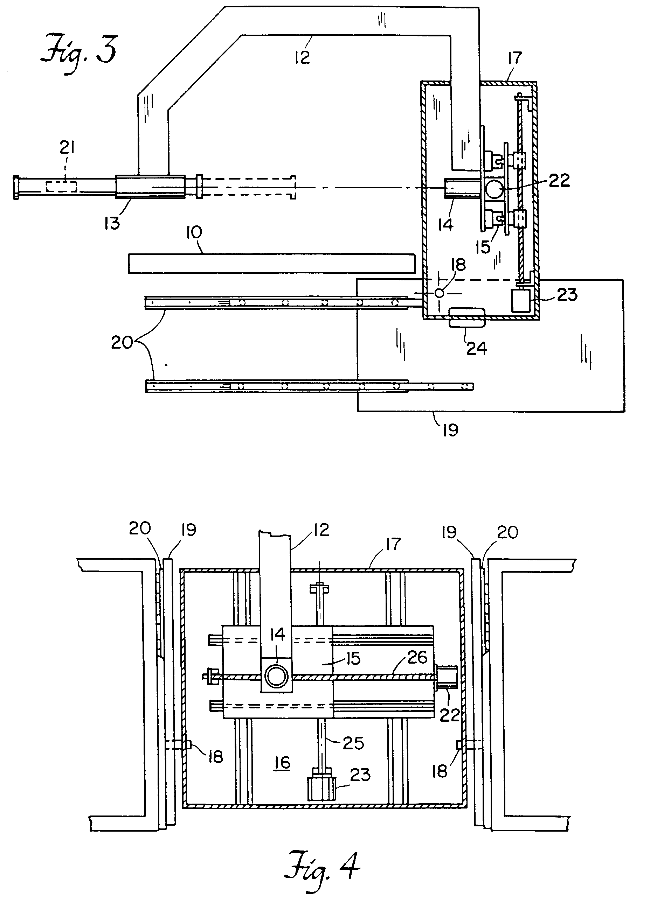

To rotate the apparatus from the anterior position shown in Figure 2 to the lateral position shown in Figure 3, the following steps must be taken. The user releases a locking mechanism and pulls the arm horizontally to one side of the table so that the saddle 17 and plates 19 slide the source from under the center of the table to avoid contact with the table during rotation. In one embodiment of the invention the source is approximately 2.5cm (one inch) below the table during anterior scanning and thus cannot be rotated without lateral movement. Source proximity to the table is desirable, as the source and detector are preferably as close to one another as possible to yield the best possible image. The drawer assembly plates 19 telescope out along the glides 20 until the pivot point 18 is astride the table 10. The plates 19 are then locked in position by a locking mechanism (not shown). The arm 12 and the attached source and saddle assembly 17 are rotated about the axis 18 to the desired position. Note that the pivot axis location must be chosen so that the source and scanning apparatus are rotated into a position just above the plane of the table. This insures that objects positioned on the table can be fully scanned laterally. The pivot location also affects the adjustment of the center of gravity as discussed below. According to the invention, the rotation is automatically controlled by adding the necessary motor and control systems; the lateral movement of the drawer assembly may likewise be automatically controlled.

Figures 2 and 3 also illustrate the presence of weights 21 and 24. After initial assembly of the apparatus, the center of gravity of the rotating elements must be adjusted to assure ease of manual rotation. In a preferred embodiment of the invention, the center of gravity of the rotating elements is located along the pivot axis 18. When the center of gravity is so situated the rotating elements will not accelerate under their own weight when the bracket 12 is rotated to any chosen angle, stopped and released.

Figure 4 shows a top view of the drawer assembly 11 and illustrates the location of the pivot axis 18, the glides 20 for displacement of the plates 19, and the tracks 26 on which the platform 15 rides. The platform 15, as well as the attached source 14, bracket 12, and detector 13, are moved in a plane perpendicular to the source-detector axis. The driving mechanism for the scanning motion is a so-called "x-y" table 16. The scanning mechanism is comprised of threaded bars, one running along the longitudinal or "y" axis 26 of the table, the second 25 running perpendicular to the first across the width or "x" axis of the table. The platform 15 has threaded housings which receive, and are driven by, the two threaded bars. The threaded "x" bar 25 is rotated by a motor 23 and the threaded "y" bar 26 is rotated by the motor 22. When the scanning assembly is rotated along with the source and detector, this insures full scanning capability at any angle. In the invention, the scanning mechanism is controlled automatically by feeding the scanning rate and the size of the area to be scanned into a computer, which then triggers the radiation source and coordinates the desired scan.

During initial rotation of the system from the vertical position, the weight of the saddle and enclosed elements controls the balancing of the bracket 12 and the attached components. The weights 24 are added to the front wall of the saddle to adjust the center of gravity in the horizontal plane. The weight 21 is added to the detector system to adjust the center of gravity in the vertical plane. As the system is rotated through larger angles from the vertical (e.g. 45°-90°), the correct weighting of the bracket and detector by weight 21 becomes more important to maintain ease of manual rotation.

By rotating the detector arm, scanning of the lumbar spine in both the anterior and lateral projections is now possible without repositioning the patient. The patient remaining in the supine position for both the lateral and anterior-posterior projection maintains the correct alignment of both projections, permits direct correlation of the two studies, and anatomically is diagnostically correct.

Performing the lateral image as the first study may enable the physician to observe extra-osseous calcification in tissue overlying the lumbar spine. In the anterior-posterior projections, such extra-osseous calcification cannot be distinguished from bone, and could therefore interfere with accurate bone density measurements in that projection. The bone being studied may be examined in real time by amplifying the signal output from the detector and displaying it on a C-T screen.

## Claims

1. Apparatus for measuring bone density in the spinal column of a subject comprising:
a stationary support (10) for supporting the subject and positioning the spinal column of the subject;
a radiation source (14) which emits radiation including two energies;
a detector (13) for detecting radiation emitted from the source to measure radiation transmitted through the subject at the two energies;
a C-shaped member (12) attached to the source (14) and detector (13) such that the source is rigidly aligned with the detector by the C-shaped member;
a drive mechanism (22) for causing relative displacement between said support (10) and the C-shaped member (12) in a direction along the spinal column of the subject to carry out a first scan of a region of the spinal column with radiation detected with said detector, said first scan being at a first angle of the member (12) relative to the support (10), a control system including a computer to control scan operation and a display screen to display an image of the scanned region; characterised by
a motor to rotate the C-shaped member (12), source (14) and detector (13), the motor being actuated by said control system to provide rotation of the C-shaped member from the first angle to the second angle such that the source and detector can be rotated in the plane about the spinal column to the second angle of the member (12) relative to the support (10) which is different from the first angle, the drive mechanism (22) causing displacement of the member (12) relative to the support (10) in a direction along the spinal column of the subject to carry out a second scan of the same region of the spinal column with radiation detected with the detector, said second scan being at said second angle of the member (12) relative to the support (10).

2. Apparatus as claimed in claim 1 wherein said motor rotates the member (12) about a rotational
axis that is translatable horizontally to displace the source (14) to one side of the spinal column to provide clearance between the support (10) and the member (12).

3. Apparatus as claimed in Claim 1 or Claim 2 wherein said source and detector are rigidly mounted on the opposite extremities of the C-shaped member.

4. Apparatus as claimed in Claim 3 wherein the C-shaped member (12) has a first end and a second end and one of said ends of said C-shaped member (12) lies closer to the support (10) than the other end.

5. Apparatus as claimed in any preceding claim wherein the distance between the radiation source (14) and said detector (13) is adjustable.

6. Apparatus as claimed in any preceding claim wherein said drive mechanism permits scanning to provide an anterior/posterior projection and a lateral projection of the spinal column.

7. Apparatus as claimed in any preceding claim in which said member (12) is mounted upon an X-Y table to enable movement of said structure in two directions.

8. Apparatus as claimed in any preceding claim in which said member (12) is movable horizontally relative to the support (10) to provide transverse motion in a direction transverse to the spinal column of the subject.

9. Apparatus as claimed in any preceding claim wherein said mechanism (22) is mounted in a drawer (11) such that said member (12) is rotatable about a horizontal axis within the drawer (11) and wherein said drawer (11) is displaced using telescoping rails (19, 20) in a direction perpendicular to the horizontal axis.

10. Apparatus as claimed in Claim 9 further comprising a balance element (21, 24) to balance said member (12) and the attached source (14) and detector (13) during rotation; and wherein said balance element (21, 24) approximately locates the center of gravity of the drawer (11), member (12), source (14) and detector (13) on the horizontal axis.

11. Apparatus as claimed in Claim 10 wherein said balance element (21, 24) is further comprised of:
a first balance element (21) distributed adjacent the source (14); and
a second balance element (24) distributed adjacent the detector (13).

12. Apparatus as claimed in any preceding claim wherein the radiation source comprises a dual energy source.

13. Apparatus as claimed in any preceding claim further comprising a motorized control system for actuating the transverse motion of the source and detector relative to the support.

14. An apparatus as in Claim 1 in which said support (10) and member (12) are relatively movable in a transverse motion in a horizontal plane.

15. An apparatus as in Claim 14 in which the transverse relative motion comprises moving the member (12) with a motor in the horizontal plane.

16. An apparatus as in Claim 1 in which said detector (13) detects radiation at two energy levels for processing to calculate bone density.

17. An apparatus as in Claim 1 in which said member (12) and support (10) are relatively movable in a direction transverse to the spinal column of the subject.

18. A method of measuring bone density in the spinal column of a patient positioned on a stationary support surface comprising:
scanning a region of the patient's spinal column at a first angle relative to the support surface by actuating a drive mechanism coupled to a scanning assembly for displacing the scanning assembly relative to the support surface, the scanning assembly having a radiation source (14) which emits radiation including two energies and a detector (13) mounted on a C-shaped member (12) such that the detector is rigidly aligned with the source (14) to receive radiation transmitted at two energies through the region from the source (14) during scanning along an axis of the spinal column, characterised by
actuating a motor by a control system which includes a computer to rotate the C-shaped member, the radiation source (14) and the detector (13) to a second angle relative to the support surface, the source (14) directing radiation through the region of the spinal column at said second angle which is different from the first angle;
scanning the region of the spinal column by actuating the drive mechanism to displace the Scanning assembly such that the detector (13) receives radiation from the source (14) that is transmitted through said region of the spinal column at the second angle; and
determining the density of bone and forming an image within the region of the spinal column from radiation received by the detector during scanning at the first and/or second angles.

19. The method of Claim 18 wherein the rotating step further comprises actuating relative horizontal displacement between the support surface and the member (12) to provide for rotation of the radiation source and detector (13) to the second angle.

20. The method of Claim 18 further comprising forming an image of a region of the spinal column at the first or second angle.

21. The method of Claim 18 further comprising providing a C-shaped member (12) to rigidly connect the source (14) and detector (13), the C-shaped member (12) being rotated during the rotating step.

22. The method of Claim 18 wherein the scanning step comprises performing a rectilinear scan with the source (14) and detector (13).

23. The method of Claim 18 wherein the step of actuating relative displacement comprises translating the member (12), source (14) and detector (13) in a horizontal plane relative to the subject to provide clearance between the support and the rotating member (12).

24. The method of claim 23 wherein said translating comprises movement in a direction transverse to the spinal column.

25. The method of claim 18 wherein said scanning at one of the first angle and the second angle comprises an anterior or a posterior scan of a region of the spinal column and said scanning at the other one of said first angle and said second angle comprises a lateral scan of a region of the spinal column.

## Patentansprüche

1. Vorrichtung zur Messung der Knochendichte in der Wirbelsäule eines Patienten, umfassend:
einen feststehenden Träger (10) zum Tragen des Patienten und zum Positionieren der Wirbelsäule des Patienten;
eine Strahlungsquelle (14), die eine zwei Energien beinhaltende Strahlung emittiert;
einen Detektor (13) zum Erfassen der von der Quelle emittierten Strahlung, um die bei den beiden Energien durch den Patienten geleitete Strahlung zu messen;
ein C-förmiges Element (12), das an der Quelle (14) und dem Detektor (13) angebracht ist, so daß die Quelle durch das C-förmige Element starr mit dem Detektor ausgerichtet ist;
eine Antriebseinrichtung (22), um eine relative Bewegung zwischen dem Träger (10) und dem C-förmigen Element (12) in einer Richtung entlang der Wirbelsäule des Patienten zu bewirken, um eine erste Abtastung eines Bereichs der Wirbelsäule mit der vom Detektor erfaßten Strahlung auszuführen, wobei die erste Abtastung unter einem ersten Winkel des Elements (12) relativ zum Träger (10) stattfindet, wobei ein Steuersystem einen Rechner zur Steuerung des Abtastvorgangs und einen Anzeigeschirm zur Anzeige eines Bildes des abgetasteten Bereichs beinhaltet; gekennzeichnet durch einen Motor zum Drehen des C-förmigen Elements (12), der Quelle (14) und des Detektors (13), wobei der Motor vom Steuersystem betätigt wird, um eine Drehung des C-förmigen Elements aus dem ersten Winkel zum zweiten Winkel zu liefern, so daß die Quelle und der Detektor in der Ebene um die Wirbelsäule zum zweiten Winkel des Elements (12) relativ zum Träger (10) gedreht werden kann, der sich vom ersten Winkel unterscheidet, wobei die Antriebseinrichtung (22) eine Bewegung des Elements (12) relativ zum Träger (10) in einer Richtung entlang der Wirbelsäule des Patienten bewirkt, um eine zweite Abtastung desselben Bereichs der Wirbelsäule mit der mit dem Detektor erfaßten Strahlung auszuführen, wobei die zweite Abtastung unter dem zweiten Winkel des Elements (12) relativ zum Träger (10) stattfindet.

2. Vorrichtung nach Anspruch 1, bei der der Motor das Element (12) um eine Drehachse dreht, die horizontal verschiebbar ist, um die Quelle (14) zu einer Seite der Wirbelsäule zu versetzen, um einen Abstand zwischen dem Träger (10) und dem Element (12) zu schaffen.

3. Vorrichtung nach Anspruch 1 oder Anspruch 2, bei der die Quelle und der Detektor an den entgegengesetzten Enden des C-förmigen Elements starr angebracht sind.

4. Vorrichtung nach Anspruch 3, bei der das C-förmige Element (12) ein erstes Ende und ein zweites Ende aufweist und eines der Enden des C-förmigen Elements (12) sich näher beim Träger (10) befindet als das andere Ende.

5. Vorrichtung nach einem der vorhergehenden Ansprüche, bei der der Abstand zwischen der Strahlungsquelle (14) und dem Detektor (13) einstellbar ist.

6. Vorrichtung nach einem der vorhergehenden Ansprüche, bei er die Antriebseinrichtung ein Abtasten gestattet, um eine vordere/hintere Darstellung und eine seitlichen Darstellung der Wirbelsäule bereitzustellen.

7. Vorrichtung nach einem der vorhergehenden Ansprüche, bei der das Element (12) auf einem XY-Tisch angebracht ist, um eine Bewegung des Aufbaus in zwei Richtungen zu ermöglichen.

8. Vorrichtung nach einem der vorhergehenden Ansprüche, bei der das Element (12) relativ zum Träger (10) horizontal beweglich ist, um eine Querbewegung in einer Richtung quer zur Wirbelsäule des Patienten zu liefern.

9. Vorrichtung nach einem der vorhergehenden Ansprüche, bei dem die Einrichtung (22) in einem Auszug (11) angebracht ist, so daß das Element (12) in dem Auszug (11) um eine horizontale Achse drehbar ist und wobei der Auszug (11) unter Verwendung sich ineinanderschiebender Schienen (19, 20) in einer Richtung senkrecht zur horizontalen Achse verschoben wird.

10. Vorrichtung nach Anspruch 9, die weiterhin ein Ausgleichselement (21, 24) umfaßt, um das Element (12) und die daran angebrachte Quelle (14) und den Detektor (13) während der Drehung im Gleichgewicht zu halten; und wobei den Schwerpunkt des Auszugs (11), des Elements (12), der Quelle (14) und des Detektors (13) durch das Ausgleichselement (21, 24) ungefähr auf der horizontalen Achse angeordnet wird.

11. Vorrichtung nach Anspruch 10, bei der das Ausgleichselement (21, 24) weiterhin aus:
einem ersten Ausgleichselement (21), das der Quelle (14) benachbart verteilt ist; und
einem zweiten Ausgleichselement (24), das dem Detektor (13) benachbart verteilt ist,
besteht.

12. Vorrichtung nach einem der vorhergehenden Ansprüche, bei der die Strahlungsquelle eine Quelle mit zwei Energien ist.

13. Vorrichtung nach einem der vorhergehenden Ansprüche, die weiter ein motorisiertes Steuersystem zum Ingangsetzen der Querbewegung der Quelle und des Detektors relativ zum Träger umfaßt.

14. Vorrichtung nach Anspruch 1, bei der der Träger (10) und das Element (12) in einer horizontalen Ebene in einer Querbewegung relativ beweglich sind.

15. Vorrichtung nach Anspruch 14, bei der die relative Bewegung in Querrichtung das Bewegen des Elements (12) mit einem Motor in der horizontalen Ebene umfaßt.

16. Vorrichtung nach Anspruch 1, bei der der Detektor (13) eine Strahlung bei zwei Energieniveaus zur Verarbeitung zur Berechnung der Knochendichte erfaßt.

17. Vorrichtung nach Anspruch 1, bei der das Element (12) und der Träger (10) in einer Richtung quer zur Wirbelsäule des Patienten relativ beweglich sind.

18. Verfahren zur Messung der Knochendichte in der Wirbelsäule eines auf einer feststehenden Trägeroberfläche befindlichen Patienten, welches umfaßt:
das Abtasten eines Bereichs der Wirbelsäule des Patienten unter einem ersten Winkel relativ zur Trägeroberfläche durch Betätigen einer mit einer Abtastanordnung verbundenen Antriebseinrichtung zum Bewegen der Abtastanordnung relativ zur Trägeroberfläche, wobei die Abtastanordnung eine Strahlungsquelle (14), die eine zwei Energien beinhaltende Strahlung emittiert, und einen Detektor (13) umfaßt, der auf einem C-förmigen Element (12) angebracht ist, so daß der Detektor starr mit der Quelle (14) ausgerichtet ist, um die während des Abtastens entlang einer Achse der Wirbelsäule von dar Quelle (14) durch den Bereich geleitete Strahlung mit zwei Energien zu empfangen, gekennzeichnet durch
das Betätigen eines Motors durch ein Steuersystem, das einen Rechner beinhaltet, um das C-förmige Element, die Strahlungsquelle (14) und den Detektor (13) zu einem zweiten Winkel relativ zur Trägeroberfläche zu drehen, wobei die Quelle (14) die Strahlung unter dem zweiten Winkel durch den Bereich der Wirbelsäule richtet, der sich vom ersten Winkel unterscheidet;
das Abtasten des Bereichs der Wirbelsäule durch Betätigen der Antriebseinrichtung, um die Abtastanordnung zu bewegen, so daß der Detektor (13) eine Strahlung von der Quelle (14) empfängt, die unter dem zweiten Winkel durch den Bereich der Wirbelsäule geleitet wird; und
das Ermitteln der Knochendichte und das Aufbauen eines Bildes im Bereich der Wirbelsäule aus der vom Detektor während des Abtastens unter dem ersten und/oder zweiten Winkel empfangenen Strahlung.

19. Verfahren nach Anspruch 18, bei dem der Schritt des Drehens weiterhin das Ingangsetzen einer relativen horizontalen Bewegung zwischen der Trägeroberfläche und dem Element (12) umfaßt, um für eine Drehung der Strahlungsquelle und des Detektors (13) zum zweiten Winkel zu sorgen.

20. Verfahren nach Anspruch 18, das weiterhin das Aufbauen eines Bildes eines Bereichs der Wirbelsäule unter dem ersten oder zweiten Winkel umfaßt.

21. Verfahren nach Anspruch 18, das weiterhin das Bereitstellen eines C-förmigen Elements (12) umfaßt, um die Quelle (14) und den Detektor (13) starr zu verbinden, wobei das C-förmige Element (12) während des Schritts des Drehens gedreht wird.

22. Verfahren nach Anspruch 18, bei dem der Abtastschritt das Ausführen einer geradlinigen Abtastung mit der Quelle (14) und dem Detektor (13) umfaßt.

23. Verfahren nach Anspruch 18, bei dem der Schritt des Ingangsetzens der relativen Bewegung das Verschieben des Elements (12), der Quelle (14) und des Detektors (13) in einer horizontalen Ebene relativ zum Patienten umfaßt, um eine Abstand zwischen den Träger und dem sich drehenden Element (12) bereitzustellen.

24. Verfahren nach Anspruch 23, bei dem das Verschieben eine Bewegung in einer Richtung quer zur Wirbelsäule umfaßt.

25. Verfahren nach Anspruch 18, bei dem das Abtasten unter dem ersten Winkel oder dem zweiten Winkel eine vordere oder eine hintere Abtastung eines Bereichs der Wirbelsäule umfaßt und das Abtasten unter dem anderen ersten Winkel oder zweiten Winkel eine seitliche Abtastung eines Bereichs der Wirbelsäule umfaßt.

## Revendications

1. Appareil de mesure de la densité osseuse dans la colonne vertébrale d'un sujet, comprenant :
un support fixe (10) destiné à supporter le sujet et à positionner la colonne vertébrale du sujet, une source (14) d'un rayonnement gui émet un rayonnement comprenant deux énergies,
un détecteur (13) destiné à détecter le rayonnement émis par la source pour mesurer le rayonnement comprenant deux énergies transmis par le sujet,
un organe (12) en C fixé à la source (14) et au détecteur (13) de manière que la source soit alignée rigidement sur le détecteur par l'organe en C,
un mécanisme (22) d'entraînement destiné à provoguer un déplacement relatif du support (10) et de l'organe en C (12) dans la direction de la longueur de la colonne vertébrale du sujet pour l'exécution d'une première analyse d'une région de la colonne vertébrale par un rayonnement détecté avec le détecteur, la première analyse étant réalisée suivant un premier angle de l'organe (12) par rapport au support (10), un système de commande comprenant un ordinateur destiné à commander l'opération d'analyse et un écran d'affichage destiné à afficher une image de la région analysée, caractérisé par :
un moteur destiné à faire tourner l'organe en C (12), la source (14) et le détecteur (13), le moteur étant commandé par le système de commande pour assurer la rotation de l'organe en C du premier angle au second angle et que la source et le détecteur puissent tourner dans le plan autour de la colonne vertébrale vers le second angle de l'organe (12) par rapport au support (10) qui est différent du premier angle, le mécanisme d'entraînement (22) provoquant un déplacement de l'organe (12) par rapport au support (10) dans la direction de la longueur de la colonne vertébrale du sujet pour l'exécution d'une seconde analyse de la même région de la colonne vertébrale par un rayonnement détecté par le détecteur, la seconde analyse étant réalisée avec le second angle de l'organe (12) par rapport au support (10).

2. Appareil selon la revendication 1, dans lequel le moteur fait tourner l'organe (12) autour d'un axe de rotation qui peut être déplacé horizontalement en translation afin que la source (14) soit déplacée d'un côté de la colonne vertébrale et laisse un espace entre le support (10) et ledit organe (12).

3. Appareil selon la revendication 1 ou 2, dans lequel la source et le détecteur sont montés rigidement aux extrémités opposées de l'organe en C.

4. Appareil selon la revendication 3, dans lequel l'organe en C (12) possède une première extrémité et une seconde extrémité, et l'une des extrémités de l'organe en C (12) est plus proche du support (10) que l'autre extrémité.

5. Appareil selon l'une quelconque des revendications précédentes, dans lequel la distance comprise entre la source (14) du rayonnement et le détecteur (13) est réglable.

6. Appareil selon l'une quelconque des revendications précédentes, dans lequel le mécanisme d'entraînement permet l'obtention d'une projection entéropostérieure et d'une projection latérale de la colonne vertébrale au cours de l'analyse.

7. Appareil selon l'une quelconque des revendications précédentes, dans lequel ledit organe (12) est monté sur une table X-Y afin que la structure puisse être déplacée dans deux directions.

8. Appareil selon l'une quelconque des revendications précédentes, dans lequel ledit organe (12) est mobile horizontalement par rapport au support (10) pour assurer un déplacement transversal en direction transversale à la colonne vertébrale du sujet.

9. Appareil selon l'une quelconque des revendications précédentes, dans lequel ledit mécanisme (22) est monté dans un tiroir (11) de manièreque ledit organe (12) puisse tourner autour d'un axe horizontal à l'intérieur du tiroir (11), et dans lequel le tiroir (11) est déplacé à l'aide de rails télescopiques (19, 20) dans une direction perpendiculaire à l'axe horizontal.

10. Appareil selon la revendication 9, comprenant en outre un élément (21, 24) d'équilibrage dudit organe (12) et de la source (14) et du détecteur (13) qui lui sont fixés lors de la rotation, et l'élément d'équilibrage (21, 24) situe approximativement le centre de gravité du tiroir (11), de l'organe (12), de la source (14) et du détecteur (13) sur l'axe horizontal.

11. Appareil selon la revendication 10, dans lequel l'élément d'équilibrage (21, 24) comporte en outre :
un premier élément d'équilibrage (21) placé près de la source (14), et
un second élément d'équilibrage (24) placé près du détecteur (13).

12. Appareil selon l'une quelconque des revendications précédentes, dans lequel la source de rayonnement est une source à deux énergies.

13. Appareil selon l'une quelconque des revendications précédentes, comprenant en outre un système motorisé de commande destiné à provoquer le mouvement transversal de la source et du détecteur par rapport au support.

14. Appareil selon la revendication 1, dans lequel le support (10) et l'organe (12) sont mobiles l'un par rapport à l'autre par un mouvement transversal dans un plan horizontal.

15. Appareil selon la revendication 14, dans lequel le mouvement relatif transversal comprend le déplacement de l'organe (12) avec le moteur dans le plan horizontal.

16. Appareil selon la revendication 1, dans lequel le détecteur (13) détecte le rayonnement à deux niveaux d'énergie afin que le traitement permette le calcul de la densité osseuse.

17. Appareil selon la revendication 1, dans lequel ledit organe (12) et le support (10) sont mobiles dans direction transversale à la colonne vertébrale du sujet.

18. Procédé de mesure de la densité osseuse de la colonne vertébrale d'un patient placé sur une surface fixe de support, comprenant :
l'analyse d'une région de la colonne vertébrale du patient avec un premier angle par rapport à la surface de support par commande d'un mécanisme d'entraînement couplé à un ensemble d'analyse destiné à déplacer l'ensemble d'analyse par rapport à la surface de support, l'ensemble d'analyse ayant une source d'un rayonnement (14) qui émet un rayonnement comprenant deux énergies et un détecteur (13) monté sur un organe en C (12) afin que le détecteur soit aligné rigidement sur la source (14) et reçoive le rayonnement transmis à deux énergies à travers la région depuis la source (14) au cours de l'analyse, le long d'un axe de la colonne vertébrale, caractérisé par :
la commande d'un moteur à l'aide d'un système de commande qui comprend un ordinateur, afin que l'organe en C, la source du rayonnement (14) et le détecteur (13) tournent vers un second angle par rapport à la surface de support, la source (14) dirigeant le rayonnement à travers la région de la colonne vertébrale suivant le second angle qui est différent du premier angle,
l'analyse de la région de la colonne vertébrale par commande du mécanisme d'entraînement afin que l'ensemble d'analyse soit déplacé si bien que le détecteur (13) reçoit le rayonnement de la source (14) qui est transmis par ladite région de la colonne vertébrale suivant le second angle, et
la détermination de la densité osseuse et la formation d'une image dans la région de la colonne vertébrale à partir du rayonnement reçu par le détecteur au cours de l'analyse au premier et/ou au second angle.

19. Procédé selon la revendication 18, dans lequel l'étape d'entraînement en rotation comporte en outre la commande d'un déplacement relatif horizontal entre la surface de support et ledit organe (12) pour assurer la rotation de la source du rayonnement et du détecteur (13) jusqu'au second angle.

20. Procédé selon la revendication 18, comprenant en outre la formation d'une image d'une région de la colonne vertébrale au premier angle ou au second angle.

21. Procédé selon la revendication 18, comprenant en outre la disposition d'un organe en C (12) destiné à raccorder rigidement la source (14) et le détecteur (13), l'organe en C (12) étant entraîné en rotation pendant l'étape de rotation.

22. Procédé selon la revendication 18, dans lequel l'étape d'analyse comprend l'exécution d'une analyse rectiligne avec la source (14) et le détecteur (13).

23. Procédé selon la revendication 18, dans lequel l'étape de commande du déplacement relatif comprend le déplacement en translation de l'organe (12), de la source (14) et du détecteur (13) dans un plan horizontal par rapport au sujet afin qu'un espace soit laissé entre le support et l'organe rotatif (12).

24. Procédé selon la revendication 23, dans lequel le déplacement en translation comprend le déplacement dans une direction transversale à la colonne vertébrale.

25. Procédé selon la revendication 18, dans lequel l'analyse à l'un des premier et second angles comprend une analyse antérieure ou une analyse postérieure d'une région de la colonne vertébrale, et l'analyse à l'autre des premier et second angles comprend une analyse latérale d'une région de la colonne vertébrale.
